# EUROPEAN PATENT APPLICATION

(11) **EP 4 410 302 A1**
(43) Date of publication of application: **07.08.2024**
(21) Application number: 22876529.3
(22) Date of filing: 30.09.2022
(51) Int. Cl.: A61K 38/17, A61P 1/16

(54) **DRUG FOR TREATING FATTY LIVER AND NON-ALCOHOLIC STEATOHEPATITIS**

(30) Priority: 30.09.2021 WO PCT/JP2021/036238
(71) Applicant: OSAKA UNIVERSITY, Suita-shi Osaka 565-0871 (JP); Niigata University, Niigata-shi, Niigata 950-2181 (JP); Stemrim Inc., Ibaraki-shi, Osaka 567-0085 (JP)
(72) Inventor: SHIMBO, Takashi, Osaka, 565-0871 (JP); TERAI, Shuji, Niigata, 951-8510 (JP); TSUCHIYA, Atsunori, Niigata, 951-8510 (JP); YAMAZAKI, Takehiko, Osaka, 567-0085 (JP); TAMAI, Katsuto., Suita-shi, Osaka, 565-0871 (JP)
(74) Representative: Vossius & Partner Patentanwälte Rechtsanwälte mbB
(86) International application number: PCT/JP2022/036662
(87) International publication number: WO 2023/054666

(57) **Abstract**

The present inventors have discovered that a peptide derived from HMGB 1 can be used as a therapeutic and/or preventive agent for fatty liver, nonalcoholic steatohepatitis, and various symptoms accompanied by the fatty liver. On the basis of this discovery, the present application provides a use of the peptide derived from HMGB 1 which is different from conventional uses.

## Description

### TECHNICAL FIELD

The present application relates to a pharmaceutical composition for prevention and/or treatment of fatty liver and nonalcoholic steatohepatitis.

### BACKGROUND ART

Fatty liver diseases are caused by virus infection, alcohol, or other reasons. Other than the viruses and alcohol, many of the fatty liver diseases are caused by diabetes, dyslipidemia, and obesity/metabolic syndrome. Nonviral and nonalcoholic fatty liver and various liver diseases caused by such fatty liver are called as nonalcoholic fatty liver disease (NAFLD). Generally, the fatty liver disease is determined to be nonalcoholic when alcohol consumption of a patient is less than 30 g/day for men and less than 20 g/day for women.

Among NAFLD, a fatty liver disease accompanied with inflammation is called as nonalcoholic steatohepatitis (NASH), and this inflammation occasionally entails fibrosis. After the fibrosis progresses, the disease becomes cirrhosis. In Japan, it is reported that 20 - 30% of adults who get a medical examination have NAFLD, and 5 - 20% of them are progressed to NASH.

Currently, there are lots of medicines for treating NASH. However, many of them are drugs to inhibit the inflammation or fibrosis of the liver, and there are no medicines effective to the fatty liver itself yet.

### PRIOR ART REFERENCES

### PATENT PUBLICATIONS

Patent Publication 1: WO 2012/147470

### NON-PATENT PUBLICATIONS

Non-Patent Publication 1: Younossi ZM, Koenig AB, Abdelatif D, Fazel Y, Henry L and Wymer M. Global epidemiology of nonalcoholic fatty liver disease-Meta-analytic assessment of prevalence, incidence, and outcomes. Hepatology. 2016; 64:73-84.

### SUMMARY OF THE INVENTION

### PROBLEMS TO BE SOLVED BY THE INVENTION

The purpose of the present application is to provide a therapeutic agent and/or a preventive agent for fatty liver and nonalcoholic steatohepatitis.

### MEANS TO SOLVE THE PROBLEMS

The present inventors discovered that a peptide derived from High mobility group box 1 (HMGB 1) protein could improve not only the inflammation and fibrosis of the liver but also the fatty liver itself.

The present application provides the followings.
[1] A pharmaceutical composition for prevention and/or treatment of fatty liver, containing a substance described in any one of the following (a) to (c):
   (a) a peptide consisting of an amino acid sequence described in SEQ ID NO: 1;
   (b) a peptide consisting of an amino acid sequence having one to four amino acid substitutions, deletions, insertions, or additions in the amino acid sequence described in SEQ ID NO: 1; and
   (c) a peptide consisting of an amino acid sequence having 90% or more sequence identity to the amino acid sequence described in SEQ ID NO: 1.
[2] The pharmaceutical composition according to the above [1], wherein the composition further improves a blood lipid concentration.
[3] The pharmaceutical composition according to the above [1] or [2], wherein the composition is repeatedly administered at least once a week for two or more weeks.
[4] The pharmaceutical composition according to any one of the above [1] to [3], wherein the fatty liver is a fatty liver of a fatty liver disease.
[5] The pharmaceutical composition according to the above [4], wherein the fatty liver disease is selected from the group consisting of nonalcoholic fatty liver disease, alcoholic fatty liver disease, nutrition-associated fatty liver disease, starvation-induced fatty liver disease, obesity-associated fatty liver disease, and diabetic fatty liver disease.
[6] A pharmaceutical composition for prevention and/or treatment of nonalcoholic steatohepatitis, containing a substance described in any one of the following (a) to (c):
   (a) a peptide consisting of an amino acid sequence described in SEQ ID NO: 1;
   (b) a peptide consisting of an amino acid sequence having one to four amino acid substitutions, deletions, insertions, or additions in the amino acid sequence described in SEQ ID NO: 1; and
   (c) a peptide consisting of an amino acid sequence having 90% or more sequence identity to the amino acid sequence described in SEQ ID NO: 1.
[7] A method of preventing and/or treating fatty liver including the step of administering to a subject in need thereof a therapeutically effective amount of a substance described in any of the following (a) to (c):
   (a) a peptide consisting of an amino acid sequence described in SEQ ID NO: 1;
   (b) a peptide consisting of an amino acid sequence having one to four amino acid substitutions, deletions, insertions, or additions in the amino acid sequence described in SEQ ID NO: 1; and
   (c) a peptide consisting of an amino acid sequence having 90% or more sequence identity to the amino acid sequence described in SEQ ID NO: 1.
[8] A substance described in any of the following (a) to (c) for use in the prevention and/or treatment of fatty liver:
   (a) a peptide consisting of an amino acid sequence described in SEQ ID NO: 1;
   (b) a peptide consisting of an amino acid sequence having one to four amino acid substitutions, deletions, insertions, or additions in the amino acid sequence described in SEQ ID NO: 1; and
   (c) a peptide consisting of an amino acid sequence having 90% or more sequence identity to the amino acid sequence described in SEQ ID NO: 1.
[9] A use of a substance described in any of the following (a) to (c) in the manufacture of a medicament for preventing and/or treating fatty liver:
   (a) a peptide consisting of an amino acid sequence described in SEQ ID NO: 1;
   (b) a peptide consisting of an amino acid sequence having one to four amino acid substitutions, deletions, insertions, or additions in the amino acid sequence described in SEQ ID NO: 1; and
   (c) a peptide consisting of an amino acid sequence having 90% or more sequence identity to the amino acid sequence described in SEQ ID NO: 1.

### BRIEF DESCRIPTION OF THE DRAWINGS

[FIG. 1] FIG. 1 is a diagram illustrating an experimental scheme of generating NASH model mice and administering the HMGB 1 fragment peptide to the mice.
[FIG. 2] FIG. 2 are graphs showing the body weight, liver weight, percentage of the liver weight to the body weight, and change of the body weight of the NASH model mice.
[FIG. 3] FIG. 3 are graphs showing the levels of aspartate aminotransferase (AST), alanine aminotransferase (ALT), albumin (Alb), total bilirubin (T-bil), total cholesterol (T-Cho), and triglyceride (TG) in the serum of the NASH model mice.
[FIG. 4] FIG. 4 are microscopic pictures showing the liver tissues of the NASH model mice (each magnification is 100 folds). From top to bottom, the pictures show the results of hematoxylin and eosin (HE) staining, F4/80 immunohistostaining, and Sirius Red staining.
[FIG. 5] FIG. 5 are graphs showing the quantified result of the Sirius Red staining, the amount of hydroxyproline, and the quantified result of the F4/80 immunohistostaining in the liver tissues of the NASH model mice.
[FIG. 6] FIG. 6 are graphs showing the inflammation of lobes of the liver tissues, ballooning of liver cells, and NAFLD Activity Score (NAS) of the NASH model mice.

### MODES FOR CARRYING OUT THE INVENTION

The present application provides a pharmaceutical composition for prevention and/or treatment of fatty liver containing a substance described in any of the following (a) to (c) (hereinafter may be referred to as "a peptide of the present application"):
(a) a peptide consisting of an amino acid sequence described in SEQ ID NO: 1;
(b) a peptide consisting of an amino acid sequence having one to four amino acid substitutions, deletions, insertions, or additions in the amino acid sequence described in SEQ ID NO: 1; and
(c) a peptide consisting of an amino acid sequence having 90% or more sequence identity to the amino acid sequence described in SEQ ID NO: 1.

The present application provides a pharmaceutical composition containing a peptide of the present application for prevention and/or treatment of nonalcoholic steatohepatitis.

The present application provides a method for prevention and/or a method for treatment of fatty liver including the step of administering to a subject in need thereof a therapeutically effective amount of the peptide of the present application. In addition, in another embodiment, a substance consisting of the peptide of the present application is provided for use in the prevention and/or treatment of fatty liver. Further, in another embodiment, a use of a substance consisting of the peptide of the present application is provided in the manufacture of a medicament for prevention and/or treatment of fatty liver.

The above mentioned invention of the present application is based on the results of the experiments and the like using an animal model having NASH induced by a high-fat diet, i.e. a NASH mouse model in which NASH is induced by feeding a high-fat diet to a melanocortin-4 receptor knockout mouse.

A "high-fat diet" in the present application is a diet containing fat preferably 20% or more as the percentage of energy. An amount of energy of such a diet is preferably 400 kcal/100 g or more. The fat contained in the high-fat diet includes, for example, animal fat such as beef tallow, lard, and milk fat, and vegetable oil such as rapeseed oil, corn oil, and soybean oil. A component other than the fat in the high-fat diet may be an ordinary dietary component, and may include a protein, a carbohydrate, a mineral and the like.

In the present application, the term "pharmaceutical composition for prevention and/or treatment" is interchangeably used with, for example, "pharmaceutical composition for use in the prevention and/or treatment" or "pharmaceutical composition for preventing and/or treating." The term "pharmaceutical composition" is interchangeably used with "medicament," "agent" or "pharmaceutic composition."

Fatty liver in the present application refers to a state in which bodies of triglyceride are accumulated in liver cells. These bodies of triglyceride are called lipid droplets, and pathologically, the state in which the lipid droplets are formed in 5% or more of the liver cells is called fatty liver. Therefore, when the percentage of the lipid droplets decreases, the fatty liver can be said to be improved. Hence, the act of decreasing the percentage of the lipid droplets is encompassed in the treatment of fatty liver in the present application.

The diagnosis criteria with regard to fatty liver are well known, and prevention and treatment of fatty liver can be evaluated based on the well-known diagnosis criteria. For example, as reported in the following reference, the degree of fatty liver can be noninvasively measured by determining Controlled Attenuation Parameter (CAP^{™}) by using Fibroscan (registered trademark). Newsome PN, Sasso M, Deeks JJ, Paredes A, Boursier J, Chan WK, Yilmaz Y, Czemichow S, Zheng MH, Wong VW, Allison M, Tsochatzis E, Anstee QM, Sheridan DA, Eddowes PJ, Guha IN, Cobbold JF, Paradis V, Bedossa P, Miette V, Foumier-Poizat C, Sandrin L, Harrison SA. FibroScan-AST (FAST) score for the non-invasive identification of patients with non-alcoholic steatohepatitis with significant activity and fibrosis: a prospective derivation and global validation study. Lancet Gastroenterol Hepatol. 2020 Apr; 5(4): 362-373.

Fatty liver in the present application includes, for example, a fatty liver of a fatty liver disease. The fatty liver disease is selected from the group consisting of nonalcoholic fatty liver disease, alcoholic fatty liver disease, nutrition-associated fatty liver disease, starvation-induced fatty liver disease, obesity-associated fatty liver disease, and diabetic fatty liver disease. A pharmaceutical composition of the present invention is effective to the various fatty liver diseases.

Nonviral or nonalcoholic fatty liver accompanied with inflammation is nonalcoholic steatohepatitis (NASH). Exactly, steatohepatitis other than the steatohepatitis caused by viral hepatitis, alcoholic hepatitis, autoimmune hepatitis, drug-induced liver disorder, and genetic disease is categorized as NASH. The inflammation of NASH may entail fibrosis. Fatty liver, inflammation, and fibrosis are three major symptoms of NASH, and pathologies such as accumulation of fat in the liver cells (lipid droplets), balloon-like swelling of the liver cells (balloon-like cells), invasion of the inflammatory cells, and fibrosis around the liver cells are usually observed in the NASH liver tissues.

Patients with NASH may have a high blood concentration of lipids such as cholesterol and triglyceride. In addition, the patients with NASH may have a liver dysfunction, and a blood concentration of a marker of the liver dysfunction such as bilirubin (Bil) may be high. Further, the patients with NASH may have a liver cell damage and a blood concentration of a marker of the liver cell damage such as AST and ALT may be high.

Therefore, a pharmaceutical composition of the present application preferably improves a blood lipid concentration of a subject. The lipid concentration used herein is, for instance, a total cholesterol concentration and a triglyceride concentration. The improvement of the blood lipid concentration used herein means, for instance, that the total cholesterol concentration decreases preferably by 20 mg/dl or more, and more preferably by 50 mg/dl or more, or the triglyceride concentration decreases preferably by 20 mg/dl or more, and more preferably by 50 mg/dl or more, after administering the pharmaceutical composition of the present application.

The term "subject" in the present application is interchangeably used with "patient," "individual" or "animal."

A peptide consisting of an amino acid sequence described in SEQ ID NO: 1 in the present application is a peptide consisting of amino acid residues 1-44 of human HMGB1 protein.

In the present application, the HMGB1 protein can be exemplified by a protein containing an amino acid sequence described in SEQ ID NO: 2 and a protein encoded by DNA containing a nucleotide sequence described in SEQ ID NO: 3, but not limited thereto.

In the pharmaceutical composition of the present application, the following peptides can be used in place of or together with the peptide consisting of an amino acid sequence described in SEQ ID NO: 1:
A) a peptide consisting of an amino acid sequence having one or more amino acid residue alterations, i.e. substitutions, deletions, insertions, or additions in the amino acid sequence described in SEQ ID NO: 1; and
B) a peptide consisting of an amino acid sequence having one or more amino acid residue alterations, i.e. substitutions, deletions, insertions, or additions in the amino acid sequence described in SEQ ID NO: 1, wherein the peptide is functionally equivalent to the peptide consisting of the amino acid sequence described in SEQ ID NO: 1.

Examples of such peptide can be exemplified by peptides described as the following i) to ii), and peptides described as the following i) to ii) which are functionally equivalent to the peptide consisting of the amino acid sequence described in SEQ ID NO: 1, but not limited thereto:
i) a peptide consisting of an amino acid sequence having one or plural, for example, 1 to 10, 1 to 9, 1 to 8, 1 to 7, 1 to 6, 1 to 5, 1 to 4, 1 to 3, or 1 or 2 amino acid substitutions, deletions, insertions, or additions in the amino acid sequence described in SEQ ID NO: 1; and
ii) a peptide consisting of an amino acid sequence having about 80% or more, for example about 85% or more, about 90% or more, about 91% or more, about 92% or more, about 93% or more, about 94% or more, about 95% or more, about 96% or more, about 97% or more, about 98% or more, or about 99% or more sequence identity to the amino acid sequence described in SEQ ID NO: 1.

In the present application, the peptide functionally equivalent to the peptide consisting of an amino acid sequence described in SEQ ID NO: 1 can be exemplified by a peptide having the following activities, but not limited thereto:
- activity which stimulates migration of a mesenchymal stem cell;
- activity which mobilizes a mesenchymal stem cell from bone marrow to blood;
- activity which mitigates inflammation;
- activity which improves fibrosis; or
- activity which regenerates a tissue.

The amino acid length of the peptide in the present application includes, for example, a range of from 25 to 35 amino acids, from 20 to 40 amino acids, from 10 to 50 amino acids, from 10 to 70 amino acids, from 10 to 100 amino acids and the like, but not limited thereto.

A therapeutically effective amount of a peptide and a pharmaceutical composition containing the same of the present application (hereinafter may be referred to as "a peptide of the present application and the like") is administered to a subject to treat or prevent diseases or symptoms described in the present specification.

The therapeutically effective amount in the present application refers to an amount sufficient to treat or prevent diseases or lesions described in the present specification. A treatment in the present application includes relief, delay, inhibition, amelioration, remission, healing, complete recovery and the like, but not limited thereto. In addition, a prevention in the present application includes relief, delay, inhibition and the like, but not limited thereto.

A subject in the present application is not particularly limited and includes mammals, birds, fish, and the like. The mammals include human or non-human animals, including, for example, human, mice, rats, monkeys, pigs, dogs, rabbits, hamsters, guinea pigs, horses, sheep, whales, and the like, but not limited thereto.

A site of administration of a peptide and the like of the present application is not limited, and the peptide and the like of the present application can exhibit their effects, even if the peptide is administered to any sites, such as a site where a lesion appears or neighborhood thereof, a site different from them (a site other than them), a site away from the site where a lesion appears, a site distal to the site where a lesion appears, or a site distal and ectopic against the site where a lesion appears.

In addition, a peptide and the like of the present application can exhibit their effects, even when administered to the liver, an organ different from the liver, an organ away from the liver, an organ distal to the liver, or an organ distal and ectopic against the liver or the like.

The method for administering a peptide and the like of the present application includes oral administration or parenteral administration, and the method for parenteral administration includes an intravascular administration such as an intraarterial administration and an intravenous administration, an intramuscular administration, a subcutaneous administration, an intradermal administration, an intraperitoneal administration, a transnasal administration, a transpulmonary administration, a transdermal administration, but not limited thereto. In addition, a peptide and the like of the present application can be administered systemically or locally (e.g., subcutaneously, intradermally, epidermally, to eyeball or palpebral conjunctiva, to nasal cavity mucosa, to oral cavity and gastrointestinal mucosa, to vaginal or intrauterine mucosa, to a legion site, etc.) by an injection, for example, intravenous injection, intramuscular injection, intraperitoneal injection, subcutaneous injection or the like.

A regimen of administering a peptide and the like of the present application is, for instance, preferably a repeated administration at least once a week for two or more weeks, and more preferably a repeated administration at least twice a week for four or more weeks.

In addition, a cell secreting a peptide of the present application, a vector for gene therapy into which a DNA encoding the peptide is inserted, and a pharmaceutical composition containing the same can also be used in place of a peptide and the like of the present application.

A peptide of the present application can be obtained as a recombinant by integrating a DNA encoding the peptide into an appropriate expression system.

In addition, a peptide of the present application can be artificially synthesized. In a synthesis method of a peptide in the present application, a peptide can be chemically synthesized by methods such as a liquid phase synthesis method and a solid phase synthesis method. The solid phase synthesis method is one of the generally employed methods when peptides are chemically synthesized. Using solid phase beads of polystyrene polymer gel having a diameter of 0.1 mm or so of which surfaces are modified with amino groups, an amino acid chain is extended one by one by dehydration reaction therefrom. After an intended sequence of the peptide is obtained, the peptide is cleaved from the solid phase surface, to obtain an intended substance. By the solid phase synthesis method, it is also possible to synthesize a ribosomal peptide which is difficult to synthesize in bacteria, introduce a non-natural amino acid such as D-form and stable isotope substitutes, i.e. ²H, ¹³C, ¹⁵N and the like, introduce a heavy atom substitute, i.e. seleno amino acids such as selenomethionine, and modify a peptide and protein main chain. When a long peptide chain exceeding from 70 to 100 residues is synthesized in the solid phase method, the peptide chain can be synthesized by binding two peptide chains using a native chemical ligation method.

A peptide in the present application may be a form of a pharmaceutically acceptable salt of the peptide. The pharmaceutically acceptable salt includes hydrochloride, acetate, trifluoroacetate and the like, but not limited thereto. The peptide in the present application may be a form of a solvate of the peptide or a solvate of a pharmaceutically acceptable salt of the peptide. The solvate refers to a solute molecule having any number of solvent molecules coordinated thereto such as a hydrate, but not limited thereto.

In addition, the method for administration can be appropriately selected depending upon age and symptoms of the subject. When the peptide of the present application is administered to a human, for example, a dose within a range of from 0.5 mg to 25 mg per 1 kg body weight per single administration can be selected. Alternatively, for example, a dose within a range of from 25 to 2500 mg/body per patient can be selected. When cells that secrete the peptide of the present application or a vector for gene therapy into which a DNA encoding the peptide is inserted is administered, the peptide can be administered such that the amount of the peptide is within the above range. However, the pharmaceutical composition of the present application is not limited to these doses.

The pharmaceutical composition of the present application can be formulated in accordance with conventional methods (e.g., Remington's Pharmaceutical Science, latest edition, Mark Publishing Company, Easton, U.S.A), which may together contain pharmaceutically acceptable carriers or additives. Examples of the carriers and additives include, for example, surfactants, excipients, colorants, perfumes, preservatives, stabilizers, buffers, suspending agents, isotonic agents, binders, disintegrants, lubricants, fluidity promoters, flavoring agents, and the like, but not limited thereto, and other conventionally used carriers can be appropriately used. Specific examples can include light anhydrous silicic acid, lactose, crystalline celluloses, mannitol, starches, carmellose calcium, carmellose sodium, hydroxypropyl cellulose, hydroxypropylmethyl cellulose, polyvinyl acetal diethylaminoacetate, polyvinyl pyrrolidone, gelatin, medium-chained fatty acid triglyceride, polyoxyethylene hydrogenated castor oil 60, white sugar, carboxymethyl cellulose, corn starch, inorganic salts, and the like.

Here, all the prior art references cited in the present specification will be incorporated herein by reference.

The present invention will be further illustrated by means of the following Examples, without intending to limit the present invention to the following Examples.

### EXAMPLES

### EXAMPLE 1

### Efficacy Evaluation of HMGB 1 Fragment Peptide for NASH Model Mouse

### (1) Materials and Methods

### i) Drug Preparation

A peptide consisting of amino acid residues 1-44 (SEQ ID NO: 1) of human HMGB1 protein was chemically synthesized by a solid phase method. Hereinafter the HMGB1 fragment peptide is simply referred to as HMGB1 peptide, and is elliptically indicated as "HMGB1" in Drawings. In addition, a control group described later is elliptically indicated as "Control" in Drawings.

### ii) Preparation of NASH Model Mice

Melanocortin-4 receptor knockout mice (hereinafter also referred to as "MC4R-KO mice," genetic background: C57BL/6J) were provided by Dr. Joel K Elmquist of University of Texas, Medical Center.

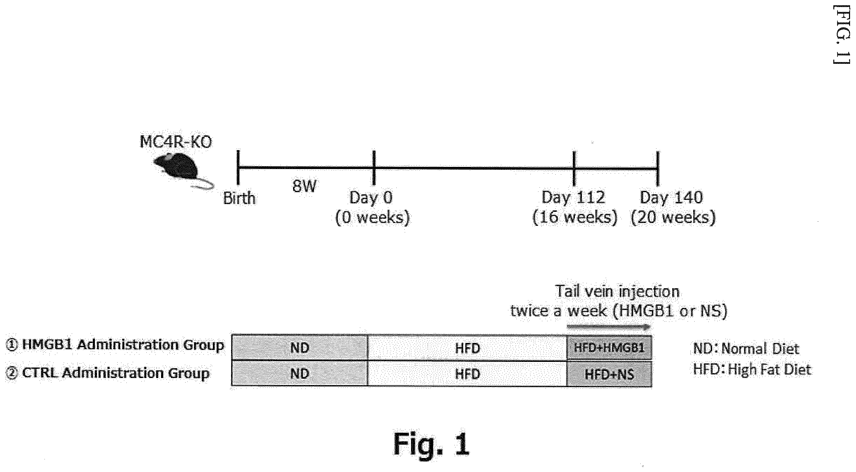

Subsequently, NASH was induced in the mice by feeding a high-fat diet to the MC4R-KO mice, as detailed in the below reference. Specifically, as indicated in Fig. 1, a normal diet (ND) was fed to the MC4R-KO mice until 8-week old. Later, the diet was switched from the normal diet to a high-fat diet (HFD) and the high-fat diet was fed for 20 weeks. Thereby, NASH was developed in the mice.
Itoh M, Suganami T, Nakagawa N, et al. Melanocortin 4 receptor-deficient mice as a novel mouse model of nonalcoholic steatohepatitis. Am J Pathol 2011; 179: 2454-63.

Here, CE-2 (manufactured by CLEA Japan, Inc.) was used as the normal diet, and Western Diet (manufactured by EPS EKISHIN Co., Ltd., containing 0.21% of cholesterol + 41 % kcal of milk fat) was used as the high-fat diet.

### iii) Administration of HMGB1 Peptide

Experiments were carried out by dividing the mice into two groups of an HMGB 1 peptide administration group (HMGB 1 administration group) and a control group (normal saline; NS administration; CTRL administration group). The HMGB1 peptide was administered with an amount of 5 mg/kg by a tail vein injection to the HMGB1 administration group twice a week for four weeks from the sixteenth week to the twentieth week after initiating the high-fat diet. The normal saline was administered with an amount of 5 ml/kg by a tail vein injection to the CTRL administration group twice a week for four weeks from the sixteenth week to the twentieth week after initiating the high-fat diet.

### iv) Efficacy Evaluation of Administration

The mice were euthanized twenty weeks after initiating the high-fat diet, and arterial blood was collected from the heart of the mice. Further, livers were collected from the mice, and the following analyses were performed on the collected blood and liver. In all of the following statistical analyses, significance was calculated by a t-test.

### a) Blood Analyses

Serum concentrations of aspartate aminotransferase (AST), alanine aminotransferase (ALT), albumin (Alb), total bilirubin (T-bil), total cholesterol (T-Cho), and triglyceride (TG) in the collected arterial blood were measured by Oriental Yeast Co., Ltd., Nagahama LSL (Nagahama, Japan).

### b) Liver Tissue Analyses

### b-1) HE Staining and Sirius Red Staining

The collected liver was fixed in 10% formalin, and sections having thickness of 4 µm were prepared. Then, hematoxylin and eosin (HE) staining and Sirius Red staining were performed to these sections. The HE-stained liver tissue sections were observed with a microscope, and the presence or absence of lipid droplets and the percentage of the lipid droplets were analyzed. In addition, ten microscopic pictures per mouse were randomly taken, and the areas stained by Sirius Red were measured, using an image analysis software.

### b-2) Immunohistostaining

The collected liver was fixed in 10% formalin, and sections having thickness of 4 µm were prepared. An immunohistostaining was performed on the sections using an anti-F4/80 rabbit monoclonal antibody (Abeam, Cambridge, UK). In the same manner as mentioned above, microscopic pictures were taken, and the areas stained with the anti-F4/80 antibody were measured, using an image analysis software.

### b-3) Hydroxyproline Assay

20 g of the collected liver tissues were homogenized, and the amount of hydroxyproline in the sample was measured, using QuickZyme Hydroxyproline Assays (QuickZyme Bioscience, Zernikedreef, Netherlands).

### b-4) Measurement of NAS

Microscopic observation was performed on the stained liver tissues obtained, and inflammation of lobes and ballooning of liver cells in the liver tissues were evaluated in accordance with the standard shown in the following Table 1. When the liver cells are damaged, the cells swell and become balloon-like. Therefore, when there are many ballooned liver cells, it means that there are many damaged liver cells.

### [Table 1]

**Table 1**

| Category | Definition | Score |
|---|---|---|
| Inflammation of lobes (Comprehensive evaluation of all the inflammatory lesions) | No lesions | 0 |
| | Number of lesions per visual field at 200 folds is less than 2 | 1 |
| | Number of lesions per visual field at 200 folds is from 2 to 4 | 2 |
| | Number of lesions per visual field at 200 folds is more than 4 | 3 |
| Ballooning of liver cells | None | 0 |
| | Few ballooned cells are found | 1 |
| | Many ballooned cells are found/Ballooning is dominant | 2 |

Further, NAFLD Activity Score (NAS) was calculated based on the below literature. NAS is an index showing the degree of pathology of NASH, and it indicates that the higher the value is, the more progressed or aggravated the pathology of NASH is.

Kleiner DE, Brunt EM, Van Natta M, Behling C, Contos MJ, Cummings OW, Ferrell LD, Liu YC, Torbenson MS, Unalp-Arida A, Yeh M, McCullough AJ, Sanyal AJ; Nonalcoholic Steatohepatitis Clinical Research Network. Design and validation of a histological scoring system for nonalcoholic fatty liver disease. Hepatology. 2005 Jun; 41(6): 1313-21.

### (2) Results

### i) Body Weight and Liver Weight

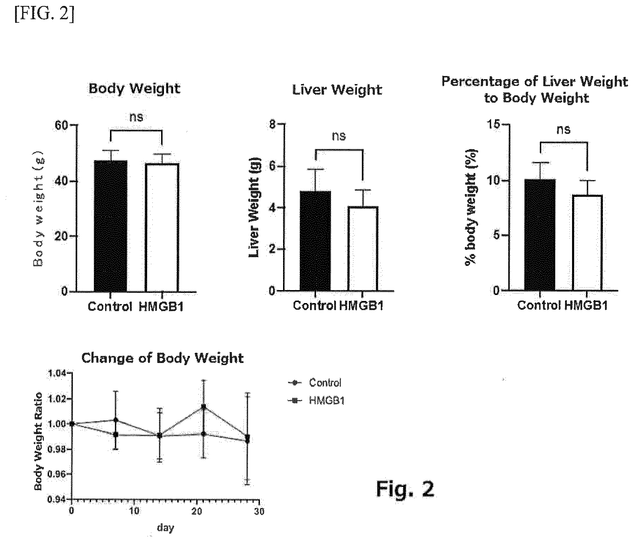

In Fig. 2, the upper left shows the body weight, the upper middle shows the liver weight, and the upper right shows the percentage of the liver weight to the body weight of the mice at the point twenty weeks after initiating the high-fat diet. In addition, the bottom of Fig. 2 indicates the change of the body weight of the mice after initiating administration of the HMGB1 peptide and until completion of breeding. As shown in these figures, significant differences were not observed in any of the body weight, liver weight, percentage of the liver weight to the body weight, and change of the body weight of the mice between the HMGB1 peptide administration group and the control group (indicated as "ns" in Figures).

### ii) Biochemical Analyses of Serum

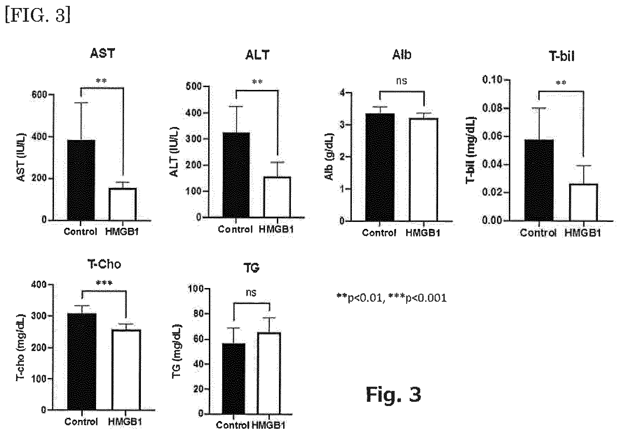

In Fig. 3, the upper left shows the AST concentration, the second left shows the ALT concentration, the second right shows the Alb concentration, and the right shows the T-bil concentration in the serum. The lower left of Fig. 3 shows the T-Cho concentration, and the lower right shows the TG concentration. The values of AST, ALT, T-bil, and T-Cho significantly decreased (improved) in the HMGB 1 peptide administration group as compared to those of the control group.

### iii) Histological Evaluation

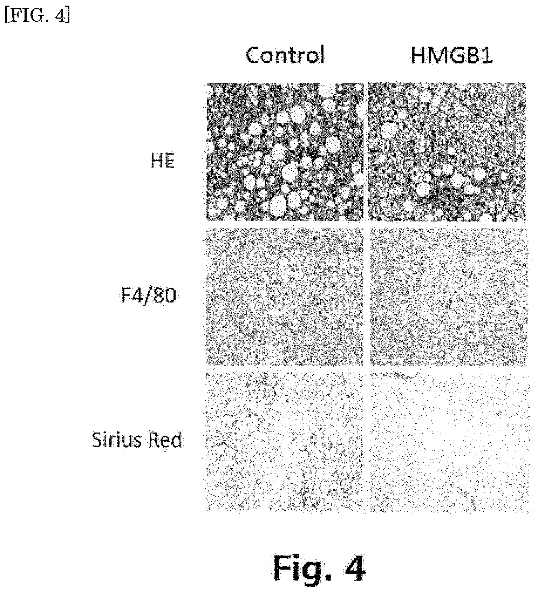

In Fig. 4, the top shows the result of the HE staining, the middle shows the result of the F4/80 immunohistostaining, and the bottom shows the result of the Sirius Red staining. On the left side are microscopic pictures of the control group, and on the right side are microscopic pictures of the HMGB 1 peptide administration group. In the HE staining, the number of lipid droplets found in the liver cells was smaller in the HMGB 1 peptide administration group than that of the control group. That is, decrease of the lipid droplets was confirmed in the HMGB 1 peptide administration group as compared to the control group. In the F4/80 immunohistostaining, the stained area and the number of F4/80 positive cells were smaller in the HMGB 1 peptide administration group than those of the control group. In the Sirius Red staining, the stained area was smaller in the HMGB 1 peptide administration group than that of the control group.

### iv) Results of Liver Tissue Analyses

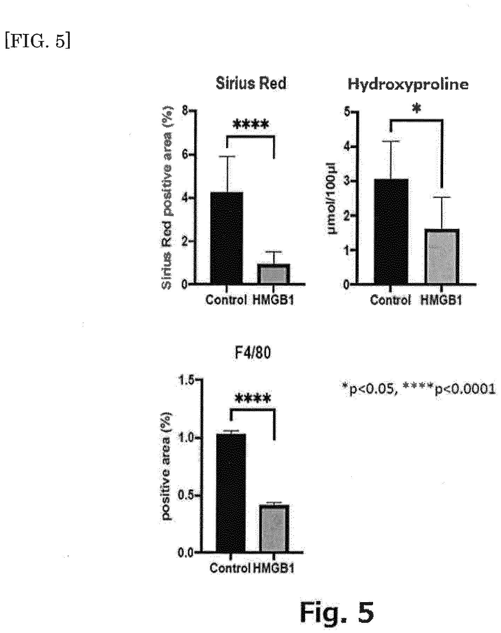

In Fig. 5, the upper left shows the Sirius Red positive area, the upper right shows the quantitative result of hydroxyproline, and the bottom shows the F4/80 positive area. The Sirius Red positive area, amount of hydroxyproline, and F4/80 positive area were all significantly lower in the HMGB 1 peptide administration group than those of the control group.

### v) Result of NAS Analysis

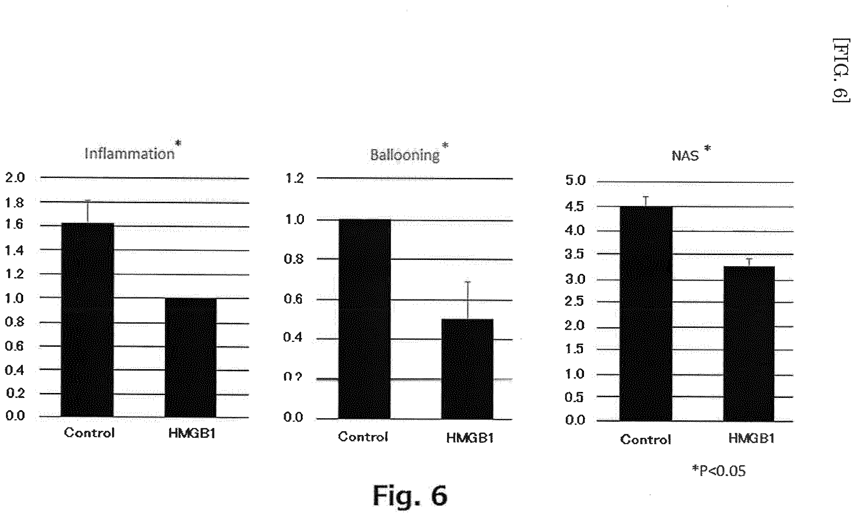

In Fig. 6, the left shows the evaluation result of the inflammation of lobes, the middle shows the evaluation result of the ballooning of liver cells, and the right shows NAS. The score of the inflammation of lobes, the score of the ballooning of liver cells, and NAS were significantly lower in the HMGB 1 peptide administration group than those of the control group.

### (3) Discussion

### i) Liver Inflammation, Liver Cell Damage and Liver

### Dysfunction

F4/80 is known to be a macrophage marker. As shown in the middle of Fig. 4 and the bottom of Fig. 5, the area stained by the F4/80 immunohistostaining and the number of F4/80 positive cells were smaller in the liver tissues of the HMGB 1 peptide administration group than those of the control group. Thus, it is suggested that in the NASH model mice, the cells which induce the inflammation were diminished by administering the HMGB 1 peptide. In addition, as shown in the left of Fig. 6, the reduction of the inflammation was observed in the HMGB 1 peptide administration group as compared to the control group in the histological evaluation. Further, as shown in the middle of Fig. 6, the score of ballooning of liver cells was significantly improved by administering the HMGB 1 peptide. Moreover, as shown in the upper left and the second left of Fig. 3, decrease of AST and ALT was observed in the biochemical analyses of the serum. Therefore, it is understood that the liver cell damage was mitigated in the HMGB1 peptide administering group as compared to the control group. In addition, as shown in the upper right of Fig. 3, decrease of T-bil was observed in the biochemical analysis of the serum. Thus, it is understood that the liver dysfunction was also mitigated in the HMGB 1 peptide administration group as compared to the control group. As a result of the above, it is considered that the HMGB 1 peptide improves the liver inflammation, liver cell damage, and liver dysfunction caused by NASH.

### ii) Fibrosis of Liver

Sirius Red is known to stain type I and type III collagen, and used as a fibrosis marker. As shown in the bottom of Fig. 4 and the upper left of Fig. 5, the area stained by Sirius Red was smaller in the liver tissues of the HMGB 1 peptide administration group than that of the control group. Thus, it is suggested that in the NASH model mice, the fibrosed part of the liver tissue was diminished by administering the HMGB 1 peptide. In addition, hydroxyproline is known to be a marker of a precursor of the fiber. The lower amount of hydroxyproline also suggests the reduction of fibrosis of the liver. As a result of the above, it is assumed that the HMGB1 peptide improves fibrosis of the liver caused by NASH.

### iii) Steatosis and Lipids in the Blood

As shown in the top of Fig. 4, the number of lipid droplets was remarkably decreased in the HMGB 1 peptide administration group as compared to that of the control group. The result indicates that the steatosis itself was improved by administering the HMGB1 peptide. Further, as shown in the lower left of Fig. 3, the T-Cho concentration was also decreased in the HMGB 1 peptide administration group as compared to the control group. This suggests that the lipid condition of the blood is also improved by administering the HMGB 1 peptide. Although many medicines for NASH improve the inflammation or fibrosis of the liver, medicines that improve the fatty liver and blood lipids are few. Thus, it is said that the HMGB1 peptide can provide the effects which are difficult to be achieved by other NASH medicines.

### INDUSTRIAL APPLICABILITY

The pharmaceutical composition containing the peptide of the present application is expected to bring great benefits that cannot be sufficiently obtained from the existing therapeutic and/or preventive agents for fatty liver, nonalcoholic steatohepatitis, and various symptoms accompanied by the fatty liver.

## Claims

1. A pharmaceutical composition for prevention and/or treatment of fatty liver, comprising a substance described in any one of the following (a) to (c):
(a) a peptide consisting of an amino acid sequence described in SEQ ID NO: 1;
(b) a peptide consisting of an amino acid sequence having one to four amino acid substitutions, deletions, insertions, or additions in the amino acid sequence described in SEQ ID NO: 1; and
(c) a peptide consisting of an amino acid sequence having 90% or more sequence identity to the amino acid sequence described in SEQ ID NO: 1.

2. The pharmaceutical composition according to claim 1, wherein the composition further improves a blood lipid concentration.

3. The pharmaceutical composition according to claim 1 or 2, wherein the composition is repeatedly administered at least once a week for two or more weeks.

4. The pharmaceutical composition according to any one of claims 1 to 3, wherein the fatty liver is a fatty liver of a fatty liver disease.

5. The pharmaceutical composition according to claim 4, wherein the fatty liver disease is selected from the group consisting of nonalcoholic fatty liver disease, alcoholic fatty liver disease, nutrition-associated fatty liver disease, starvation-induced fatty liver disease, obesity-associated fatty liver disease, and diabetic fatty liver disease.

6. A pharmaceutical composition for prevention and/or treatment of nonalcoholic steatohepatitis comprising a substance described in any one of the following (a) to (c):
(a) a peptide consisting of an amino acid sequence described in SEQ ID NO: 1;
(b) a peptide consisting of an amino acid sequence having one to four amino acid substitutions, deletions, insertions, or additions in the amino acid sequence described in SEQ ID NO: 1; and
(c) a peptide consisting of an amino acid sequence having 90% or more sequence identity to the amino acid sequence described in SEQ ID NO: 1.
